# FASCICULE DE BREVET EUROPEEN

(11) **EP 2 804 955 B1**
(45) Date de publication et mention de la délivrance du brevet: **01.11.2017**
(21) Numéro de dépôt: 12816739.2
(22) Date de dépôt: 19.12.2012
(51) Int. Cl.: C12Q 1/68

(54) **PROCEDE POUR LE DIAGNOSTIC IN VITRO DU CANCER DE L'OVAIRE**
VERFAHREN ZUR IN-VITRO-DIAGNOSE VON EIERSTOCKKREBS
A METHOD FOR THE IN VITRO DIAGNOSIS OF OVARIAN CANCER

(30) Priorité: 20.12.2011 FR 1162104
(43) Date de publication de la demande: 26.11.2014
(73) Titulaire: Biomérieux, 69280 Marcy l'Étoile (FR)
(72) Inventeur: PEROT, Philippe, F-69007 Lyon (FR); MALLET, François, F-69100 Villeurbanne (FR); MUGNIER, Nathalie, 69008 Lyon (FR)
(74) Mandataire: Agasse, Stéphane
(86) Numéro de dépôt international: PCT/FR2012/053003
(87) Numéro de publication internationale: WO 2013/093347

(56) Documents cités:
- EP-A1- 2 340 851
- WO-A2-01/70979
- WO-A2-02/079473
- WO-A2-2007/137279
- FENG WANG-JOHANNING ET AL: "Expression of multiple human endogenous retrovirus surface envelope proteins in ovarian cancer", INTERNATIONAL JOURNAL OF CANCER, vol. 120, no. 1, 29 septembre 2006 (2006-09-29), pages 81-90, XP055034826, ISSN: 0020-7136, DOI: 10.1002/ijc.22256
- J. GIMENEZ ET AL: "Custom human endogenous retroviruses dedicated microarray identifies self-induced HERV-W family elements reactivated in testicular cancer upon methylation control", NUCLEIC ACIDS RESEARCH, vol. 38, no. 7, 6 janvier 2010 (2010-01-06), pages 2229-2246, XP055055471, ISSN: 0305-1048, DOI: 10.1093/nar/gkp1214
- SEIFARTH WOLFGANG ET AL: "Comprehensive analysis of human endogenous retrovirus transcriptional activity in human tissues with a retrovirus-specific microarray", JOURNAL OF VIROLOGY, vol. 79, no. 1, janvier 2005 (2005-01), pages 341-352, XP002694975, ISSN: 0022-538X
- J. PACES: "HERVd: the Human Endogenous RetroViruses Database: update", NUCLEIC ACIDS RESEARCH, vol. 32, no. 90001, 1 janvier 2004 (2004-01-01), pages 50D-50, XP55056305, ISSN: 0305-1048, DOI: 10.1093/nar/gkh075
- DATABASE EMBL [Online] 26 avril 1999 (1999-04-26), "Homo sapiens BAC clone RP11-432D12 from 2, complete sequence.", XP002694976, extrait de EBI accession no. EM_STD:AC007319 Database accession no. AC007319
- DATABASE EMBL [Online] 15 octobre 1999 (1999-10-15), "Human DNA sequence from clone RP4-603I14 on chromosome 1p31.3-33", XP002694977, extrait de EBI accession no. EM_STD:AL122001 Database accession no. AL122001
- DATABASE EMBL [Online] 23 juillet 2007 (2007-07-23), "Homo sapiens FOSMID clone ABC9-44034100M10 from chromosome 1, complete sequence.", XP002694978, extrait de EBI accession no. EM_STD:AC207061 Database accession no. AC207061
- MENENDEZ LAURA ET AL: "L1 and HERV-W retrotransposons are hypomethylated in human ovarian carcinomas", MOLECULAR CANCER, BIOMED CENTRAL, LONDON, GB, vol. 3, no. 1, 26 avril 2004 (2004-04-26), page 12, XP021008196, ISSN: 1476-4598, DOI: 10.1186/1476-4598-3-12
- PHILIPPE PÉROT ET AL: "Microarray-Based Sketches of the HERV Transcriptome Landscape", PLOS ONE, vol. 7, no. 6, 28 juin 2012 (2012-06-28), page e40194, XP055034857, ISSN: 1932-6203, DOI: 10.1371/journal.pone.0040194
- DATABASE EMBL [Online] 13 juin 2001 (2001-06-13), "Homo sapiens chromosome 5 clone CTD-2365E22, complete sequence.", XP002698070, extrait de EBI accession no. EM_STD:AC091867 Database accession no. AC091867
- DATABASE EMBL [Online] 31 juillet 2001 (2001-07-31), "Homo sapiens chromosome 1 clone RP11-400N13, complete sequence.", XP002698071, extrait de EBI accession no. EM_STD:AC092765 Database accession no. AC092765
- DATABASE EMBL [Online] 21 février 2002 (2002-02-21), "Homo sapiens BAC clone RP13-644M16 from 4, complete sequence.", XP002698072, extrait de EBI accession no. EM_STD:AC111000 Database accession no. AC111000
- Philippe Pérot ET AL: "Microarray-Based Sketches of the HERV Transcriptome Landscape", PLoS ONE, vol. 7, no. 6, 1 January 2012 (2012-01-01) , page e40194, XP055034857, ISSN: 1932-6203, DOI: 10.1371/journal.pone.0040194
- Philippe Pérot ET AL: "Microarray-based sketches of the HERV transcriptome landscape: Supplementary Table S3", PloS one, vol. 7, no. 6, 28 June 2008 (2008-06-28), XP055356742,

## Description

Les rétrovirus endogènes constituent la descendance de rétrovirus infectieux s'étant intégrés sous leur forme provirale dans des cellules de la lignée germinale et ayant été transmis par ce biais dans le génome de la descendance de l'hôte.

Le séquençage du génome humain a permis de révéler l'extrême abondance des éléments transposables ou de leurs dérivés. De fait, les séquences répétées représentent près de la moitié du génome humain et les rétrovirus endogènes et les rétrotransposons en composent 8% avec un nombre s'élevant, à ce jour, à plus de 400 000 éléments.

L'abondance des éléments rétroviraux endogènes (ERVs) présents actuellement dans le génome humain est le résultat d'une centaine d'endogénisations réussies au cours de l'évolution de la lignée humaine. Les différentes vagues d'endogénisation s'étalent sur une période allant de 2 à 90 millions d'années avant notre ère et ont été suivies de l'expansion du nombre de copies par des phénomènes de type « copier/coller » avec possibilité d'apparition d'erreurs, conduisant à partir d'un provirus ancestral à la formation d'une famille de HERV, c'est à dire un ensemble d'éléments présentant des homologies de séquences. Les plus anciens éléments, ceux de la famille HERV-L, se seraient intégrés avant l'émergence des mammifères. Deux familles HERV-F et HERV-H sont apparues dans la période où les premiers primates faisaient leur apparition. Les familles HERV-FRD et HERV-K(HML-5) intégrées il y a 40 à 55 millions d'années, sont spécifiques des primates supérieurs. En revanche les familles HERV-W et HERV-E, par exemple, se sont intégrées 5 à 10 millions d'années plus tard après la séparation avec les singes du nouveau monde, et sont spécifiques des Catarhini (Hominoides et Cercopithèques).

Les séquences ERVs sont représentées sur l'ensemble des chromosomes, avec une densité variant selon les familles et il n'existe pas de corrélation entre la proximité physique des ERVs et leur proximité phylogénétique.

Les ERVs ont longtemps été considérés comme des parasites ou comme de simples déchets de l'ADN. Néanmoins, l'impact des ERVs sur l'organisme n'est pas uniquement limité à leur participation passée dans le modelage du génome ou à des recombinaisons délétères pouvant encore subvenir.

L'abondance et la complexité structurelle des ERVs rendent les analyses de leur expression très compliquées et souvent difficilement interprétables. La détection de l'expression de HERV peut refléter l'activation transcriptionnelle de un ou de plusieurs loci au sein d'une même famille. Le ou les loci activés peuvent de plus varier en fonction du tissu et/ou du contexte.
Les présents inventeurs ont maintenant découvert et démontré, que des séquences d'acides nucléiques correspondant à des loci précisément identifiés d'éléments rétroviraux endogènes, sont associés au cancer de l'ovaire et que ces séquences sont des marqueurs moléculaires de la pathologie. Les séquences identifiées sont soit des provirus, c'est à dire des séquences contenant tout ou partie des gènes gag, pol et env flanqués en 5' et 3' de longues terminaisons répétées (LTR ou « Long Terminal Repeat » selon la terminologie anglo-saxonne), soit tout ou partie des LTR ou des gènes isolés. Les séquences ADN identifiées sont respectivement référencées en SEQ ID NO : 1 à 510 dans le listage de séquences, leur localisation chromosomique est identifiée dans le tableau ci-dessous (NCBI 36/hgl8), ainsi que leur expression, surexpression ou sous-expression représentées par le « ratio d'expression » entre échantillon cancéreux et échantillon normal. Quand l'expression de l'acide nucléique ou le changement d'expression de l'acide nucléique est spécifique du tissu ovaire on indique cette information par le symbole « x » dans la colonne tissu cible. Ceci signifie que si une expression ou un changement d'expression de l'acide nucléique concerné est déterminé dans un compartiment biologique autre que le tissu ovaire, ceci représente, à distance, une signature d'un cancer de l'ovaire. Les séquences ADN identifiées comme étant spécifiques du tissu ovaire sont respectivement référencées en SEQ ID NOs : SEQ ID NO: 2, 3, 6, 9, 10, 11, 20, 23, 24, 25, 26, 30, 34, 59 et 139. Les séquences ADN identifiées comme étant non spécifiques du tissu ovaire sont respectivement référencées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510.

**Tableau**

| SEQ ID NO: | Localisation chromosomique | Tissu cible | Ratio d'expression cancer/normal |
|---|---|---|---|
| 1 | (-) chr 1: 204451670-204454441 | | 23,6 |
| 2 | (-) chr 5: 31145123-31145616 | x | 20,6 |
| 3 | (-) chr 1: 220215255-220218922 | x | 18,9 |
| 4 | (+) chr 20: 15876573-15877028 | | 15,3 |
| 5 | (-) chr 10: 85918238-85921075 | | 13,9 |
| 6 | (+) chr 4: 69952306-69955060 | x | 10,7 |
| 7 | (-) chr 7: 136602660-136602962 | | 9,4 |
| 8 | (-) chr 19: 21408391-21408433 | | -9,3 |
| 9 | (+) chr 5: 138886787-138888726 | x | 8,7 |
| 10 | (+) chr 1: 53662597-53668291 | x | 8,2 |
| 11 | (-) chr 11: 61892539-61895047 | x | 6,8 |
| 12 | (-) chr 6: 31258778-31261514 | | 6,8 |
| 13 | (-) chr 4: 92495874-92498563 | | 6,2 |
| 14 | (-) chr 17: 72119645-72120398 | | 6,2 |
| 15 | (+) chr 13: 108715439-108721465 | | 5,8 |
| 16 | (-) chr 4: 92408723-92409131 | | 5,3 |
| 17 | (+) chr 2: 188084458-188084785 | | -5,1 |
| 18 | (+) chr 1: 201726731-201727141 | | -5,1 |
| 19 | (-) chr 20: 15911118-15913833 | | 4,8 |
| 20 | (-) chr 2: 34879568-34885219 | x | 4,5 |
| 21 | (-) chr 3: 32480101-32483411 | | 4,3 |
| 22 | (-) chr 1: 20229120-20230095 | | -4,0 |
| 23 | (-) chr 3: 32477433-32480101 | x | 3,9 |
| 24 | (-) chr X: 135840667-135841473 | x | 3,7 |
| 25 | (-) chr 6: 27901601-27902447 | x | 3,7 |
| 26 | (-) chr 7: 120206619-120210175 | x | 3,7 |
| 27 | (-) chr 2: 54587807-54590183 | | -3,6 |
| 28 | (+) chr 11: 302654-302777 | | -3,6 |
| 29 | (-) chr 1: 46569499-46569788 | | 3,5 |
| 30 | (+) chr 13: 79655647-79662117 | x | 3,5 |
| 31 | (+) chr 2: 223763483-223764405 | | -3,2 |
| 32 | (-) chr 21: 16686151-16692725 | | -3,2 |
| 33 | (+) chr 7: 22344951-22345519 | | 3,2 |
| 34 | (-) chr 1: 77133084-77138660 | x | 2,9 |
| 35 | (-) chr 3: 5525407-5525762 | | 2,9 |
| 36 | (+) chr 19: 5797895-5801213 | | 2,8 |
| 37 | (+) chr 5: 162693944-162700167 | | 2,8 |
| 38 | (+) chr 22: 22208249-22219093 | | 2,7 |
| 39 | (-) chr 11: 123241237-123246982 | | 2,7 |
| 40 | (+) chr X: 18907929-18908893 | | 2,6 |
| 41 | (+) chr 8: 116896352-116900252 | | 2,6 |
| 42 | (+) chr 1: 46568022-46568774 | | 2,5 |
| 43 | (-) chr 3: 44361156-44367804 | | 2,5 |
| 44 | (-) chr 12: 123151029-123151568 | | 2,5 |
| 45 | (-) chr 3: 171872658-171878745 | | -2,5 |
| 46 | (-) chr 18: 24526989-24532843 | | 2,4 |
| 47 | (-) chr 4: 190639686-190643551 | | 2,4 |
| 48 | (-) chr 3: 172018098-172024244 | | -2,4 |
| 49 | (+) chr 5: 109894536-109899114 | | 2,4 |
| 50 | (+) chr 9: 139130635-139131502 | | 2,4 |
| 51 | (-) chr 17: 59750387-59751101 | | -2,4 |
| 52 | (-) chr 2: 63928103-63928550 | | -2,3 |
| 53 | (-) chr 1: 23201713-23201784 | | -2,3 |
| 54 | (-) chr X: 119100721-119100753 | | 2,3 |
| 55 | (+) chr 1: 38146074-38152175 | | -2,3 |
| 56 | (+) chr 6: 131686129-131689771 | | -2,3 |
| 57 | (+) chr 2: 34868390-34874629 | | 2,2 |
| 58 | (-) chr X: 119100721-119100753 | | 2,2 |
| 59 | (+) chr 2: 188606470-188612115 | x | 2,2 |
| 60 | (-) chr 3: 149764167-149767943 | | -2,2 |
| 61 | (-) chr X: 16107244-16112478 | | 2,1 |
| 62 | (+) chr 16: 20680398-20681069 | | -2,1 |
| 63 | (-) chr 17: 16678919-16679896 | | -2,1 |
| 64 | (+) chr 4: 53395726-53396112 | | 2,1 |
| 65 | (-) chr 19: 58630272-58630359 | | 2,1 |
| 66 | (+) chr 14: 22268133-22268207 | | -2,0 |
| 67 | (+) chr 16: 29617077-29617563 | | 2,0 |
| 68 | (+) chr 7: 93107183-93112802 | | 2,0 |
| 69 | (-) chr 1: 226879232-226884835 | | -2,0 |
| 70 | (-) chr 7: 97418554-97422641 | | 2,0 |
| 71 | (+) chr 2: 175898117-175899503 | | -2,0 |
| 72 | (-) chr X: 153489882-153497212 | | -2,0 |
| 73 | (-) chr 12: 10006301-10012556 | | 2,0 |
| 74 | (+) chr 17: 49777998-49778501 | | 2,0 |
| 75 | (-) chr 10: 62463482-62469677 | | 2,0 |
| 76 | (+) chr 7: 63784637-63784717 | | 1,9 |
| 77 | (-) chr 9: 22648524-22654061 | | -1,9 |
| 78 | (-) chr 19: 41338217-41343765 | | 1,9 |
| 79 | (+) chr 3: 75269085-75276706 | | -1,9 |
| 80 | (+) chr 14: 50429630-50433350 | | 1,9 |
| 81 | (+) chr 3: 113681517-113681938 | | 1,9 |
| 82 | (+) chr 17: 18280923-18281897 | | -1,9 |
| 83 | (+) chr 17: 30852752-30853047 | | -1,9 |
| 84 | (-) chr 7: 16683220-16684043 | | 1,9 |
| 85 | (-) chr 17: 72113663-72119188 | | 1,9 |
| 86 | (-) chr 6: 66439380-66440579 | | 1,9 |
| 87 | (+) chr 14: 49582825-49583215 | | 1,9 |
| 88 | (-) chr 3: 146311752-146313676 | | 1,9 |
| 89 | (-) chr 6: 82110364-82117596 | | -1,9 |
| 90 | (-) chr 16: 8834700-8846815 | | 1,8 |
| 91 | (-) chr 3: 83646334-83646729 | | -1,8 |
| 92 | (+) chr 2: 57102769-57103266 | | 1,8 |
| 93 | (-) chr 9: 87683968-87685669 | | 1,8 |
| 94 | (+) chr 17: 7905950-7906340 | | 1,8 |
| 95 | (-) chr 1: 79726879-79732396 | | 1,8 |
| 96 | (+) chr X: 154839291-154839392 | | 1,8 |
| 97 | (-) chr 1: 20240363-20244851 | | -1,8 |
| 98 | (-) chr 19: 51297295-51305072 | | 1,8 |
| 99 | (-) chr 1: 89419377-89419778 | | -1,8 |
| 100 | (+) chr 13: 61603944-61604298 | | 1,8 |
| 101 | (-) chr 5: 81601427-81604174 | | 1,8 |
| 102 | (-) chr 11: 61899593-61907139 | | 1,8 |
| 103 | (+) chr 1: 237429952-237430254 | | -1,8 |
| 104 | (+) chr 19: 23052386-23053407 | | 1,8 |
| 105 | (+) chr 4: 149285541-149286218 | | -1,8 |
| 106 | (+) chr 13: 78118938-78119911 | | 1,8 |
| 107 | (+) chr 1: 46558555-46559522 | | 1,8 |
| 108 | (-) chr X: 107838-108808 | | 1,8 |
| 109 | (-) chr 6: 5981329-5981780 | | 1,8 |
| 110 | (+) chr 5: 79443327-79444232 | | 1,7 |
| 111 | (+) chr 3: 102893427-102902549 | | 1,7 |
| 112 | (+) chr 17: 70760297-70765658 | | 1,7 |
| 113 | (-) chr 3: 130842785-130843595 | | 1,7 |
| 114 | (-) chr 14: 45033906-45038685 | | 1,7 |
| 115 | (+) chr 7: 63778493-63778556 | | 1,7 |
| 116 | (+) chr 5: 130936343-130941430 | | 1,7 |
| 117 | (+) chr 15: 88477019-88482534 | | -1,7 |
| 118 | (+) chr 2: 30592323-30596634 | | -1,7 |
| 119 | (+) chr 3: 100621292-100621816 | | -1,7 |
| 120 | (+) chr 3: 28017061-28017725 | | -1,7 |
| 121 | (+) chr 4: 121134267-121140271 | | 1,7 |
| 122 | (-) chr 1: 113164281-113170199 | | 1,7 |
| 123 | (+) chr 2: 231645891-231645949 | | 1,7 |
| 124 | (-) chr 20: 23684500-23685884 | | -1,7 |
| 125 | (+) chr 21: 41248565-41252290 | | 1,7 |
| 126 | (-) chr 7: 26030345-26035880 | | 1,7 |
| 127 | (-) chr 1: 82866215-82866932 | | 1,7 |
| 128 | (-) chr 19: 36769982-36771248 | | 1,7 |
| 129 | (-) chr 8: 99671725-99672015 | | 1,7 |
| 130 | (-) chr 21: 45534498-45534683 | | -1,7 |
| 131 | (+) chr 8: 72739454-72745239 | | 1,7 |
| 132 | (+) chr 1: 171710187-171711158 | | 1,7 |
| 133 | (-) chr 4: 80695843-80699887 | | -1,7 |
| 134 | (-) chr 20: 15076339-15082148 | | 1,7 |
| 135 | (-) chr 18: 26600808-26601241 | | -1,6 |
| 136 | (-) chr 19: 48519503-48525376 | | 1,6 |
| 137 | (+) chr 4: 184621848-184622846 | | -1,6 |
| 138 | (-) chr 5: 118311697-118316179 | | 1,6 |
| 139 | (+) chr 20: 825581-830240 | x | 1,6 |
| 140 | (-) chr X: 73258716-73266192 | | -1,6 |
| 141 | (-) chr 6: 137423130-137429951 | | -1,6 |
| 142 | (-) chr 19: 43813619-43814569 | | 1,6 |
| 143 | (-) chr 6: 166679549-166680132 | | 1,6 |
| 144 | (-) chr 3: 170131404-170133251 | | -1,6 |
| 145 | (-) chr 9: 73768007-73768097 | | -1,6 |
| 146 | (-) chr 19: 42435136-42435718 | | 1,6 |
| 147 | (+) chr 3: 127091992-127101129 | | -1,6 |
| 148 | (-) chr 3: 70566511-70572260 | | 1,6 |
| 149 | (+) chr 7: 134507781-134510522 | | 1,6 |
| 150 | (+) chr 1: 101404629-101404696 | | -1,6 |
| 151 | (+) chr 19: 63026807-63027775 | | 1,6 |
| 152 | (-) chr 20: 740491-741481 | | 1,6 |
| 153 | (+) chr 10: 100097920-100098685 | | -1,6 |
| 154 | (+) chr 4: 87614948-87620699 | | 1,6 |
| 155 | (-) chr 3: 188666779-188672482 | | 1,6 |
| 156 | (-) chr 12: 8805277-8812326 | | -1,6 |
| 157 | (-) chr 1: 119033717-119034387 | | 1,6 |
| 158 | (-) chr 2: 194703669-194709523 | | 1,6 |
| 159 | (-) chr 7: 53274415-53274795 | | 1,6 |
| 160 | (+) chr 19: 60146916-60147844 | | 1,6 |
| 161 | (+) chr 9: 21911892-21921934 | | 1,6 |
| 162 | (+) chr 17: 18280923-18281897 | | -1,6 |
| 163 | (+) chr X: 3737016-3737408 | | -1,6 |
| 164 | (-) chr 3: 186765708-186772209 | | 1,6 |
| 165 | (-) chr X: 64265907-64271621 | | 1,6 |
| 166 | (-) chr 21: 43094024-43099757 | | 1,6 |
| 167 | (-) chr Y: 4283432-4289271 | | 1,6 |
| 168 | (+) chr 1: 227127533-227128140 | | 1,6 |
| 169 | (+) chr 19: 58638840-58639824 | | 1,6 |
| 170 | (+) chr 1: 55260969-55266632 | | 1,6 |
| 171 | (-) chr 6: 161967666-161968129 | | 1,5 |
| 172 | (-) chr 18: 8170226-8170996 | | -1,5 |
| 173 | (-) chr 17: 64303192-64305325 | | -1,5 |
| 174 | (-) chr 4: 139016243-139016901 | | 1,5 |
| 175 | (-) chr 9: 90136987-90137726 | | 1,5 |
| 176 | (+) chr 16: 84869202-84872386 | | 1,5 |
| 177 | (+) chr 18: 69142830-69148577 | | 1,5 |
| 178 | (-) chr 14: 38837505-38837576 | | 1,5 |
| 179 | (-) chr 5: 164658658-164659366 | | 1,5 |
| 180 | (+) chr 4: 16529457-16529865 | | 1,5 |
| 181 | (+) chr 14: 58981836-58986258 | | 1,5 |
| 182 | (+) chr 11: 20545997-20550777 | | 1,5 |
| 183 | (+) chr 7: 24405964-24408131 | | 1,5 |
| 184 | (+) chr 8: 98282174-98288062 | | -1,5 |
| 185 | (-) chr 15: 51506333-51511952 | | 1,5 |
| 186 | (+) chr 17: 57579448-57580087 | | 1,5 |
| 187 | (+) chr 2: 173115073-173117251 | | 1,5 |
| 188 | (-) chr 14: 100841342-100847337 | | -1,5 |
| 189 | (-) chr 1: 223094264-223100407 | | 1,5 |
| 190 | (+) chr 19: 42188901-42189862 | | -1,5 |
| 191 | (-) chr 1: 110071319-110071749 | | 1,5 |
| 192 | (+) chr 11: 71138116-71141391 | | -1,5 |
| 193 | (+) chr 17: 11815798-11820989 | | 1,5 |
| 194 | (-) chr 21: 26522067-26527677 | | 1,5 |
| 195 | (-) chr 13: 110287593-110288562 | | -1,5 |
| 196 | (-) chr 12: 14769094-14769447 | | -1,5 |
| 197 | (-) chr 8: 828875-829287 | | 1,5 |
| 198 | (+) chr 2: 57307944-57315854 | | -1,5 |
| 199 | (+) chr 15: 72433538-72439180 | | -1,5 |
| 200 | (-) chr 20: 30839204-30839675 | | 1,5 |
| 201 | (+) chr 6: 121287670-121293704 | | 1,5 |
| 202 | (-) chr 5: 92818136-92819135 | | -1,5 |
| 203 | (-) chr 2: 80956433-80960797 | | 1,5 |
| 204 | (+) chr 3: 133114616-133115281 | | 1,5 |
| 205 | (+) chr 15: 93094982-93104611 | | 1,5 |
| 206 | (-) chr 17: 41716757-41717724 | | 1,5 |
| 207 | (-) chr 1: 208103713-208104413 | | 1,5 |
| 208 | (+) chr 7: 20126857-20130993 | | 1,5 |
| 209 | (-) chr 5: 152811649-152815183 | | -1,5 |
| 210 | (+) chr 21: 19397535-19398503 | | 1,5 |
| 211 | (-) chr 1: 205981124-205981888 | | 1,5 |
| 212 | (-) chr 13: 63016887-63017674 | | -1,5 |
| 213 | (-) chr 2: 40978866-40978933 | | 1,5 |
| 214 | (-) chr 6: 123945178-123950851 | | 1,5 |
| 215 | (-) chr 12: 20983776-20984452 | | 1,5 |
| 216 | (-) chr 4: 41854992-41855740 | | 1,5 |
| 217 | (+) chr 19: 57816195-57826383 | | 1,5 |
| 218 | (+) chr 3: 198173309-198174307 | | 1,5 |
| 219 | (-) chr 4: 5101984-5102366 | | 1,5 |
| 220 | (-) chr 11: 42988611-42989260 | | 1,5 |
| 221 | (-) chr 10: 53463521-53469327 | | -1,5 |
| 222 | (+) chr 2: 215375861-215376821 | | 1,5 |
| 223 | (-) chr 1: 39742864-39743840 | | -1,5 |
| 224 | (+) chr 2: 49358270-49358629 | | -1,5 |
| 225 | (+) chr 3: 37222224-37226355 | | 1,5 |
| 226 | (-) chr 6: 11215545-11220435 | | 1,5 |
| 227 | (-) chr 4: 177452380-177455182 | | 1,4 |
| 228 | (-) chr 3: 193273756-193277341 | | 1,4 |
| 229 | (+) chr 13: 35788462-35794330 | | -1,4 |
| 230 | (+) chr 8: 90837193-90837630 | | 1,4 |
| 231 | (-) chr 6: 126064685-126070602 | | 1,4 |
| 232 | (-) chr 9: 92557313-92561369 | | 1,4 |
| 233 | (+) chr 13: 34271392-34275485 | | 1,4 |
| 234 | (-) chr 2: 188741658-188747663 | | -1,4 |
| 235 | (+) chr 6: 131658190-131664031 | | 1,4 |
| 236 | (-) chr 4: 11264289-11269976 | | 1,4 |
| 237 | (-) chr 3: 167701085-167706900 | | 1,4 |
| 238 | (-) chr 8: 34837245-34837699 | | 1,4 |
| 239 | (-) chr X: 84258326-84258441 | | 1,4 |
| 240 | (-) chr 1: 230032039-230038017 | | -1,4 |
| 241 | (-) chr 1: 1340045-1340674 | | 1,4 |
| 242 | (-) chr 11: 23865384-23871043 | | 1,4 |
| 243 | (+) chr 15: 53797305-53798112 | | 1,4 |
| 244 | (+) chr 8: 105367316-105373215 | | 1,4 |
| 245 | (+) chr 6: 36585121-36585596 | | 1,4 |
| 246 | (-) chr 3: 180722967-180726830 | | -1,4 |
| 247 | (+) chr 1: 113153812-113159470 | | 1,4 |
| 248 | (+) chr 6: 123203066-123208413 | | -1,4 |
| 249 | (+) chr 13: 40927858-40928571 | | 1,4 |
| 250 | (+) chr 2: 151518177-151527698 | | -1,4 |
| 251 | (+) chr 4: 125955713-125956405 | | 1,4 |
| 252 | (-) chr 10: 6879049-6884921 | | -1,4 |
| 253 | (-) chr 2: 38161446-38167158 | | 1,4 |
| 254 | (+) chr 5: 115014416-115019003 | | 1,4 |
| 255 | (-) chr 6: 96115220-96116183 | | 1,4 |
| 256 | (+) chr 4: 105081874-105087636 | | 1,4 |
| 257 | (-) chr 4: 77184473-77184590 | | 1,4 |
| 258 | (+) chr 1: 237331217-237334832 | | 1,4 |
| 259 | (+) chr 5: 129737615-129737869 | | 1,4 |
| 260 | (+) chr 10: 72056196-72056562 | | 1,4 |
| 261 | (+) chr 11: 70894199-70894610 | | 1,4 |
| 262 | (-) chr 22: 37782215-37788928 | | -1,4 |
| 263 | (-) chr 19: 60153464-60154429 | | 1,4 |
| 264 | (-) chr 20: 25478990-25479969 | | -1,4 |
| 265 | (+) chr 12: 101073296-101074047 | | 1,4 |
| 266 | (+) chr X: 119059748-119060186 | | -1,4 |
| 267 | (+) chr 6: 66335631-66336029 | | -1,4 |
| 268 | (-) chr X: 48298854-48299852 | | 1,4 |
| 269 | (-) chr 6: 111714710-111717380 | | 1,4 |
| 270 | (+) chr 15: 79410960-79411606 | | -1,4 |
| 271 | (-) chr 4: 20240490-20246699 | | 1,4 |
| 272 | (-) chr 8: 7800551-7804236 | | -1,4 |
| 273 | (-) chr 12: 84386849-84387812 | | -1,4 |
| 274 | (+) chr 1: 237334832-237336987 | | -1,4 |
| 275 | (-) chr 13: 22285789-22286759 | | 1,4 |
| 276 | (+) chr 8: 8677169-8677775 | | 1,4 |
| 277 | (-) chr 3: 134664420-134665419 | | 1,4 |
| 278 | (+) chr 2: 88760597-88762729 | | -1,4 |
| 279 | (+) chr 18: 71806397-71806963 | | 1,4 |
| 280 | (+) chr 6: 123199111-123199590 | | 1,4 |
| 281 | (+) chr 6: 63559535-63565316 | | 1,4 |
| 282 | (-) chr X: 8364892-8365592 | | 1,4 |
| 283 | (-) chr 11: 69581214-69582655 | | 1,4 |
| 284 | (-) chr 16: 57560802-57562053 | | 1,4 |
| 285 | (-) chr 1: 15334421-15335379 | | 1,4 |
| 286 | (+) chr 3: 8130557-8130971 | | -1,4 |
| 287 | (-) chr 6: 80623291-80623866 | | 1,4 |
| 288 | (+) chr 14: 42343224-42346079 | | 1,4 |
| 289 | (-) chr 16: 21140609-21141570 | | 1,4 |
| 290 | (-) chr 7: 146321019-146321687 | | -1,4 |
| 291 | (-) chr 11: 92482373-92482685 | | 1,4 |
| 292 | (+) chr 19: 20126744-20133095 | | 1,4 |
| 293 | (+) chr 4: 44685507-44693742 | | 1,4 |
| 294 | (+) chr 13: 54525767-54533878 | | -1,4 |
| 295 | (-) chr 2: 77980822-77981234 | | -1,4 |
| 296 | (-) chr 19: 59483969-59484458 | | 1,4 |
| 297 | (+) chr 18: 13646676-13647837 | | 1,4 |
| 298 | (+) chr 3: 187581597-187582311 | | 1,4 |
| 299 | (-) chr 18: 65578866-65580015 | | 1,4 |
| 300 | (+) chr 13: 81344919-81345826 | | -1,4 |
| 301 | (+) chr 9: 12938344-12944129 | | -1,4 |
| 302 | (+) chr X: 119059748-119060186 | | -1,4 |
| 303 | (+) chr 2: 18474209-18474872 | | -1,4 |
| 304 | (+) chr 7: 66718910-66719589 | | 1,4 |
| 305 | (-) chr 21: 27028460-27028873 | | -1,4 |
| 306 | (-) chr 2: 101601994-101602661 | | 1,4 |
| 307 | (-) chr 4: 59057092-59057870 | | -1,4 |
| 308 | (+) chr 7: 100353142-100354585 | | 1,4 |
| 309 | (+) chr 11: 58524024-58529760 | | 1,4 |
| 310 | (+) chr 3: 46140179-46140255 | | 1,4 |
| 311 | (-) chr X: 112238600-112244308 | | 1,4 |
| 312 | (+) chr 9: 34993290-34999332 | | 1,4 |
| 313 | (-) chr 10: 92051390-92052344 | | -1,4 |
| 314 | (+) chr 19: 23528001-23529694 | | 1,4 |
| 315 | (-) chr 10: 23719462-23720038 | | 1,3 |
| 316 | (-) chr 9: 15409320-15410036 | | 1,3 |
| 317 | (+) chr 10: 13383338-13383595 | | -1,3 |
| 318 | (+) chr 16: 56798947-56799017 | | 1,3 |
| 319 | (-) chr 15: 19500293-19500872 | | 1,3 |
| 320 | (+) chr 17: 19640286-19640712 | | -1,3 |
| 321 | (-) chr 4: 135818540-135824157 | | 1,3 |
| 322 | (+) chr 5: 108685641-108685853 | | -1,3 |
| 323 | (+) chr 3: 83648234-83648933 | | 1,3 |
| 324 | (-) chr 1: 210389154-210391126 | | 1,3 |
| 325 | (-) chr 1: 36727172-36729315 | | -1,3 |
| 326 | (+) chr 3: 133850331-133851314 | | -1,3 |
| 327 | (+) chr 2: 71468737-71476455 | | -1,3 |
| 328 | (-) chr 2: 69523645-69529977 | | -1,3 |
| 329 | (-) chr 19: 20128147-20129118 | | 1,3 |
| 330 | (+) chr 3: 153797495-153803634 | | 1,3 |
| 331 | (-) chr X: 107838-108808 | | 1,3 |
| 332 | (+) chr 1: 65378067-65379033 | | -1,3 |
| 333 | (-) chr 4: 138339335-138340015 | | 1,3 |
| 334 | (-) chr 6: 68639470-68641070 | | 1,3 |
| 335 | (-) chr 16: 69214387-69217522 | | 1,3 |
| 336 | (-) chr 1: 205873717-205874167 | | 1,3 |
| 337 | (-) chr Y: 12999559-12999968 | | -1,3 |
| 338 | (-) chr 10: 32280890-32281905 | | -1,3 |
| 339 | (-) chr 3: 68563582-68564080 | | -1,3 |
| 340 | (+) chr 4: 8472992-8480379 | | 1,3 |
| 341 | (-) chr 7: 27747804-27748772 | | -1,3 |
| 342 | (+) chr 8: 72749726-72755451 | | 1,3 |
| 343 | (-) chr 9: 116976750-116982362 | | 1,3 |
| 344 | (-) chr 2: 202508160-202508613 | | 1,3 |
| 345 | (+) chr 16: 10294212-10296592 | | 1,3 |
| 346 | (-) chr 2: 105967906-105968229 | | 1,3 |
| 347 | (-) chr 2: 98027450-98028453 | | 1,3 |
| 348 | (+) chr 7: 100357831-100363586 | | 1,3 |
| 349 | (+) chr 8: 98256433-98262143 | | 1,3 |
| 350 | (+) chr 9: 79145953-79146072 | | 1,3 |
| 351 | (-) chr 7: 128961001-128961758 | | 1,3 |
| 352 | (-) chr Y: 14976683-14981289 | | -1,3 |
| 353 | (-) chr 13: 88856202-88856780 | | -1,3 |
| 354 | (+) chr 1: 204107496-204110550 | | -1,3 |
| 355 | (+) chr 2: 170676773-170684540 | | 1,3 |
| 356 | (-) chr 5: 37820710-37821012 | | -1,3 |
| 357 | (+) chr 3: 40577391-40583175 | | 1,3 |
| 358 | (+) chr 15: 33670273-33670510 | | 1,3 |
| 359 | (-) chr 1: 194451065-194451430 | | 1,3 |
| 360 | (+) chr 21: 18019170-18019257 | | 1,3 |
| 361 | (-) chr 6: 24784895-24791307 | | 1,3 |
| 362 | (+) chr 4: 8486531-8489137 | | -1,3 |
| 363 | (+) chr 1: 4781688-4782412 | | -1,3 |
| 364 | (-) chr 7: 48049968-48050752 | | 1,3 |
| 365 | (+) chr 22: 31228741-31234490 | | 1,3 |
| 366 | (+) chr 6: 149002601-149008339 | | -1,3 |
| 367 | (-) chr 11: 67377518-67381008 | | 1,3 |
| 368 | (+) chr 1: 70826708-70832264 | | -1,3 |
| 369 | (-) chr 7: 149362156-149371533 | | 1,3 |
| 370 | (+) chr 3: 191345083-191348529 | | 1,3 |
| 371 | (+) chr 4: 157740798-157746638 | | -1,3 |
| 372 | (+) chr 2: 199752333-199758641 | | -1,3 |
| 373 | (+) chr 6: 130555149-130561062 | | -1,3 |
| 374 | (+) chr 8: 7247222-7250906 | | -1,3 |
| 375 | (-) chr 2: 58194071-58199769 | | 1,3 |
| 376 | (+) chr 18: 46153554-46153955 | | 1,3 |
| 377 | (+) chr 5: 43015565-43018176 | | 1,3 |
| 378 | (-) chr 1: 20280815-20281721 | | -1,3 |
| 379 | (-) chr 7: 52362517-52362663 | | 1,3 |
| 380 | (+) chr 19: 17565375-17569904 | | 1,3 |
| 381 | (+) chr 12: 30100281-30106718 | | -1,3 |
| 382 | (-) chr 15: 39364817-39365176 | | -1,3 |
| 383 | (-) chr 3: 163923299-163927462 | | 1,3 |
| 384 | (-) chr 3: 48735692-48742987 | | -1,3 |
| 385 | (-) chr 3: 9864346-9867038 | | 1,3 |
| 386 | (+) chr 21: 44461476-44462142 | | 1,3 |
| 387 | (+) chr 12: 34314239-34318461 | | 1,3 |
| 388 | (-) chr 5: 18704707-18710464 | | 1,3 |
| 389 | (-) chr 7: 39726352-39726804 | | 1,3 |
| 390 | (+) chr 7: 35702274-35703153 | | 1,3 |
| 391 | (-) chr 8: 136848614-136854437 | | 1,3 |
| 392 | (-) chr 20: 54220244-54221269 | | -1,3 |
| 393 | (+) chr 4: 151308190-151308663 | | 1,3 |
| 394 | (+) chr 2: 127843921-127850615 | | -1,3 |
| 395 | (+) chr 3: 186986788-186987217 | | 1,3 |
| 396 | (-) chr X: 72941188-72945945 | | -1,3 |
| 397 | (-) chr 12: 66446853-66452393 | | -1,3 |
| 398 | (+) chr 7: 82926920-82932637 | | -1,3 |
| 399 | (-) chr 18: 38386402-38392105 | | -1,3 |
| 400 | (+) chr 2: 209475743-209478438 | | -1,3 |
| 401 | (+) chr 15: 81168183-81174208 | | -1,3 |
| 402 | (-) chr 20: 23789232-23790195 | | 1,3 |
| 403 | (+) chr 13: 55144122-55144740 | | -1,3 |
| 404 | (+) chr X: 138586657-138587350 | | -1,2 |
| 405 | (+) chr 1: 227155887-227156667 | | -1,2 |
| 406 | (+) chr 4: 68579259-68585888 | | 1,2 |
| 407 | (-) chr X: 137174181-137180160 | | -1,2 |
| 408 | (+) chr X: 30031651-30037293 | | 1,2 |
| 409 | (+) chr X: 153791448-153792743 | | -1,2 |
| 410 | (-) chr 12: 33132053-33135058 | | -1,2 |
| 411 | (-) chr 15: 18488924-18489503 | | 1,2 |
| 412 | (+) chr 2: 179035945-179036517 | | 1,2 |
| 413 | (+) chr 5: 43093994-43094958 | | -1,2 |
| 414 | (-) chr 11: 67365951-67369209 | | 1,2 |
| 415 | (-) chr 2: 187305381-187306066 | | -1,2 |
| 416 | (+) chr 19: 58554156-58559856 | | 1,2 |
| 417 | (+) chr 2: 36679747-36685410 | | -1,2 |
| 418 | (+) chr 3: 143021313-143023045 | | 1,2 |
| 419 | (-) chr 8: 56851123-56851350 | | 1,2 |
| 420 | (+) chr 1: 207713890-207714591 | | 1,2 |
| 421 | (-) chr 1: 201241191-201241627 | | 1,2 |
| 422 | (+) chr 3: 116946112-116948448 | | -1,2 |
| 423 | (-) chr 6: 78424455-78432877 | | -1,2 |
| 424 | (-) chr 10: 86314943-86315696 | | 1,2 |
| 425 | (+) chr X: 115379535-115382006 | | -1,2 |
| 426 | (+) chr 18: 49536517-49537209 | | 1,2 |
| 427 | (-) chr Y: 13181166-13187309 | | -1,2 |
| 428 | (-) chr 10: 9461231-9461724 | | 1,2 |
| 429 | (-) chr 13: 83508263-83508914 | | 1,2 |
| 430 | (-) chr 5: 121980485-121987968 | | 1,2 |
| 431 | (-) chr 12: 91257229-91258011 | | 1,2 |
| 432 | (-) chr 3: 170033623-170034359 | | 1,2 |
| 433 | (-) chr 15: 22481598-22487267 | | -1,2 |
| 434 | (+) chr 15: 50517982-50518757 | | -1,2 |
| 435 | (+) chr 3: 147554294-147559942 | | -1,2 |
| 436 | (+) chr 8: 120998753-120999678 | | -1,2 |
| 437 | (+) chr 3: 78353823-78354204 | | 1,2 |
| 438 | (-) chr 20: 3962724-3963629 | | -1,2 |
| 439 | (+) chr 16: 54225830-54226967 | | -1,2 |
| 440 | (-) chr 10: 52210114-52210776 | | -1,2 |
| 441 | (+) chr 5: 98593423-98593800 | | -1,2 |
| 442 | (-) chr 21: 41718929-41719369 | | 1,2 |
| 443 | (+) chr 4: 146973214-146979106 | | 1,2 |
| 444 | (-) chr X: 148516015-148516993 | | 1,2 |
| 445 | (+) chr 1: 161827723-161832945 | | 1,2 |
| 446 | (-) chr 14: 24964620-24969975 | | -1,2 |
| 447 | (+) chr 11: 321893-322552 | | -1,2 |
| 448 | (-) chr X: 89718079-89718557 | | -1,2 |
| 449 | (+) chr 6: 119115236-119116007 | | -1,2 |
| 450 | (-) chr 2: 44099248-44105173 | | 1,2 |
| 451 | (-) chr 18: 1990815-1991782 | | 1,2 |
| 452 | (+) chr 10: 65936583-65936972 | | 1,2 |
| 453 | (-) chr 11: 77247021-77252169 | | 1,2 |
| 454 | (-) chr 12: 45932745-45933508 | | 1,2 |
| 455 | (-) chr 12: 17764451-17768938 | | 1,2 |
| 456 | (+) chr 11: 6343834-6349468 | | -1,2 |
| 457 | (+) chr 13: 43180036-43180733 | | 1,2 |
| 458 | (+) chr 7: 65695119-65695525 | | 1,2 |
| 459 | (-) chr 5: 146727162-146727562 | | -1,2 |
| 460 | (+) chr 14: 46643150-46647720 | | 1,2 |
| 461 | (-) chr 2: 101341556-101342578 | | 1,2 |
| 462 | (-) chr 1: 209462097-209463065 | | 1,2 |
| 463 | (+) chr 14: 25196411-25196706 | | -1,2 |
| 464 | (-) chr 13: 19604221-19604442 | | -1,2 |
| 465 | (-) chr 1: 71574420-71575497 | | 1,2 |
| 466 | (-) chr 3: 144273854-144274284 | | 1,2 |
| 467 | (-) chr 7: 68382377-68383085 | | -1,2 |
| 468 | (-) chr 18: 68050597-68051144 | | 1,2 |
| 469 | (+) chr 11: 67283732-67289415 | | 1,2 |
| 470 | (+) chr 3: 90214824-90218570 | | -1,2 |
| 471 | (-) chr 5: 135094585-135101074 | | 1,2 |
| 472 | (+) chr 4: 175540733-175541728 | | -1,2 |
| 473 | (-) chr 2: 47832401-47832875 | | -1,2 |
| 474 | (+) chr 11: 104640788-104641691 | | -1,2 |
| 475 | (-) chr 12: 20862483-20866818 | | 1,2 |
| 476 | (-) chr 9: 138794598-138798686 | | -1,2 |
| 477 | (+) chr 1: 45554293-45558755 | | -1,2 |
| 478 | (+) chr 12: 122598918-122609265 | | 1,2 |
| 479 | (-) chr 2: 231220016-231220435 | | 1,2 |
| 480 | (-) chr 19: 57024203-57024671 | | 1,2 |
| 481 | (+) chr 21: 14114072-14115040 | | 1,2 |
| 482 | (+) chr 8: 81814506-81817982 | | -1,2 |
| 483 | (+) chr 13: 62685327-62691268 | | -1,2 |
| 484 | (+) chr 3: 163912942-163918742 | | 1,1 |
| 485 | (-) chr 6: 111682993-111683951 | | -1,1 |
| 486 | (+) chr 7: 85150987-85151764 | | 1,1 |
| 487 | (+) chr 6: 131841491-131842465 | | 1,1 |
| 488 | (-) chr 1: 144213068-144214036 | | -1,1 |
| 489 | (+) chr 2: 201439950-201440437 | | -1,1 |
| 490 | (+) chr 9: 129215427-129217168 | | -1,1 |
| 491 | (+) chr 14: 99217652-99218668 | | 1,1 |
| 492 | (+) chr 12: 28874497-28874581 | | 1,1 |
| 493 | (+) chr 3: 95139589-95145594 | | -1,1 |
| 494 | (+) chr 8: 91057690-91058157 | | 1,1 |
| 495 | (+) chr 17: 31491551-31492522 | | -1,1 |
| 496 | (-) chr 2: 167330763-167331232 | | 1,1 |
| 497 | (-) chr 3: 100178327-100178459 | | -1,1 |
| 498 | (-) chr 2: 239567148-239569048 | | -1,1 |
| 499 | (+) chr 18: 27623185-27623837 | | -1,1 |
| 500 | (+) chr 4: 177503741-177503879 | | 1,1 |
| 501 | (+) chr 15: 52966922-52970766 | | -1,1 |
| 502 | (-) chr 4: 8535024-8541336 | | -1,1 |
| 503 | (+) chr 3: 38150975-38151716 | | -1,1 |
| 504 | (+) chr 10: 104955188-104963400 | | 1,1 |
| 505 | (+) chr 3: 42827687-42828671 | | 1,1 |
| 506 | (+) chr 19: 60921086-60922051 | | 1,1 |
| 507 | (+) chr 12: 60267154-60272981 | | -1,1 |
| 508 | (+) chr 18: 23066222-23066669 | | 1,1 |
| 509 | (-) chr 18: 31472178-31472680 | | -1,1 |
| 510 | (-) chr 4: 190637685-190639587 | | 1,1 |

La présente invention a donc pour objet un procédé pour le diagnostic, *in vitro,* du cancer de l'ovaire, *in vitro,* du cancer de l'ovaire dans un échantillon biologique prélevé chez un patient, qui comprend une étape de détection d'au moins un produit d'expression d'au moins une séquence d'acide nucléique, ladite séquence d'acide nucléique étant choisie parmi les séquences identifiées en SEQ ID NOs : 2, 3, 6, 9, 10, 11, 20, 23, 24, 25, 26, 30, 34, 59 et 139 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs :2, 3, 6, 9, 10, 11, 20, 23, 24, 25, 26, 30, 34, 59 et 139.

Le diagnostic permet d'établir si un individu est malade ou non malade. Le pronostic permet d'établir un degré de gravité de la maladie (grades et/ou stades) qui a une incidence sur la survie et/ou qualité de vie de l'individu. Dans le cadre de la présente invention, le diagnostic peut être très précoce.

Le pourcentage d'identité décrit ci-dessus a été déterminé en prenant en considération la diversité nucléotidique dans le génome. Il est connu que la diversité nucléotidique est plus élevée dans les régions du génome riches en séquences répétées que dans les régions ne contenant pas de séquences répétées. A titre d'exemple, Nickerson D. A. et al. (1) ont montré une diversité d'environ 0,3% (0,32%) dans des régions contenant des séquences répétées. Selon l'invention, les séquences qui présentent au moins 99% d'identité, présentent de préférence au moins 99,5% d'identité, avantageusement au moins 99,6 % ou au moins 99,7% d'identité.

La capacité de discrimination d'un état cancéreux de chacune des séquences identifiées ci-dessus a été mise en évidence à l'aide d'une analyse statistique utilisant la procédure SAM (5) suivie d'une correction par le taux de faux positif (6) et d'une suppression des valeurs inférieures à 2⁶. Par conséquent chacune des séquences identifiées ci-dessus présente une différence d'expression significative entre un état tumoral et un état normal. Il en résulte qu'une différence d'expression observée pour une des séquences précitées constitue une signature de la pathologie. Bien entendu, il est possible de combiner les différences d'expression relevées pour plusieurs des séquences référencées ci-dessus par exemple par une ou des associations de 2, 3, 4, 5, 6, 7, 8, 9, 10 et plus jusqu'à 510 des séquences listées. En particulier les séquences identifiées en SEQ ID NOs : 1, 17 et 29, prises en combinaison (2 à 2 ou 3) avec les séquences SEQ ID NOs: 2, 3, 6, 9, 10, 11, 20, 23, 24, 25, 26, 30, 34, 59 et 139 constituent une ou des signatures privilégiées.

Dans un mode de réalisation du procédé selon l'invention on détecte le produit d'expression d'au moins deux séquences d'acide nucléique, lesdites au moins deux séquences d'acide nucléique étant
choisies parmi les séquences identifiées comme étant spécifiques du tissu ovaire, c'est à dire choisies dans le groupe de séquences identifiées en SEQ ID NOs : 2, 3, 6, 9, 10, 11, 20, 23, 24, 25, 26, 30, 34, 59 et 139, en particulier les séquences choisies parmi les séquences identifiées en SEQ ID NOs : 2, 3 et 6 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 2, 3, 6, 9, 10, 11, 20, 23, 24, 25, 26, 30, 34, 59 et 139, en particulier parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 2, 3 et 6.

Dans encore un autre mode de réalisation de l'invention, on détecte le produit d'expression d'au moins une séquence choisie parmi les séquences identifiées comme étant spécifiques du tissu ovaire, c'est à dire choisie dans le groupe des séquences identifiées en SEQ ID NOs : 2, 3, 6, 9, 10, 11, 20, 23, 24, 25, 26, 30, 34, 59 et 139 ou choisie parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 2, 3, 6, 9, 10, 11, 20, 23, 24, 25, 26, 30, 34, 59 et 139 et le produit d'expression d'au moins une séquence choisie parmi les séquences identifiées comme étant non spécifiques du tissu ovaire, c'est à dire choisie dans le groupe de séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510, en particulier les séquences choisies parmi les séquences identifiées en SEQ ID NOs 1, 17 et 19, ou choisie parmi les séquences qui présentent au moins 99% d'identité, de préférence au moins 99,7% d'identité et avantageusement au moins 99,6% ou au moins 99,7% d'identité avec une des séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510, en particulier parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 17 et 19.

Le produit d'expression détecté est au moins un transcrit ARN, en particulier au moins un ARNm ou au moins un polypeptide.

Lorsque le produit d'expression est un transcrit ARNm, il est détecté par toute méthode appropriée, telle que l'hybridation, le séquençage ou l'amplification. L'ARNm peut être détecté directement par mise en contact avec au moins une sonde et/ou au moins une amorce qui sont conçues pour s'hybrider dans des conditions expérimentales prédéterminées aux transcrits ARNm, la mise en évidence de la présence ou de l'absence d'hybridation à l'ARNm et éventuellement la quantification de l'ARNm. Parmi les méthodes préférées on peut citer l'amplification (par exemple la RT-PCR, la NASBA, etc.), l'hybridation sur puce ou encore le séquençage. L'ARNm peut également être détecté indirectement à partir d'acides nucléiques dérivés desdits transcrits, comme les copies ADNc, etc.

Généralement le procédé de l'invention comprend une étape initiale d'extraction de l'ARNm de l'échantillon à analyser.

Ainsi, le procédé peut comprendre :
(i) une étape d'extraction de l'ARNm de l'échantillon à analyser,
(ii) une étape de détection et de quantification de l'ARNm de l'échantillon à analyser,
(iii) une étape d'extraction de l'ARNm dans un échantillon de référence, qui peut être un échantillon sain et provenant du même individu ou,
(iv) une étape de détection et de quantification de l'ARNm de l'échantillon sain,
(iii) une étape de comparaison de la quantité d'ARNm exprimé dans l'échantillon à analyser et dans l'échantillon de référence ; la détermination d'une quantité d'ARNm exprimé dans l'échantillon à analyser différente de la quantité d'ARNm exprimé dans l'échantillon de référence sain pouvant être corrélée avec le diagnostic d'un cancer de l'ovaire (la différence de la quantité d'ARNm dans le tissu ovaire cancéreux par rapport à la quantité d'ARNm exprimé dans le tissu ovaire sain étant indifféremment une expression, une surexpression ou une sous-expression);
et en particulier :
(i) une extraction de l'ARNm à analyser de l'échantillon,
(ii) une détermination, dans l'ARN à analyser, d'un niveau d'expression d'au moins une séquence ARN dans l'échantillon, ladite séquence ARN étant le produit de transcription d'au moins une séquence d'acide nucléique choisie parmi les séquences identifiées en SEQ ID NOs : 2, 3, 6, 9, 10, 11, 20, 23, 24, 25, 26, 30, 34, 59 et 139 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 2, 3, 6, 9, 10, 11, 20, 23, 24, 25, 26, 30, 34, 59 et 139, et
(iii) une comparaison du niveau d'expression de la ou des séquence(s) ARN définie(s) en (ii) avec un niveau d'expression de référence ; la détermination d'un niveau d'expression de l'ARN à analyser présentant une différence par rapport au niveau d'expression de référence pouvant être corrélé avec le diagnostic d'un cancer de l'ovaire (comme déterminé ci-dessus) ; ou
(i) une étape d'extraction de l'ARNm de l'échantillon à analyser,
(ii) une étape de détection et de quantification de l'ARNm de l'échantillon à analyser,
(iii) une étape de comparaison de la quantité d'ARNm exprimé dans l'échantillon à analyser par rapport à une quantité d'ARNm de référence, la détermination d'une quantité d'ARNm exprimé dans l'échantillon à analyser différente de la quantité d'ARNm de référence pouvant être corrélée avec le diagnostic d'un cancer de l'ovaire (la différence de la quantité d'ARNm dans l'échantillon à analyser par rapport à la quantité d'ARNm de référence étant indifféremment une expression, une surexpression ou une sous-expression).

Dans un mode de réalisation du procédé de l'invention on prépare des copies ADN de l'ARNm, on met en contact les copies ADN avec au moins une sonde et/ou au moins une amorce dans des conditions prédéterminées permettant l'hybridation et en ce qu'on détecte la présence ou l'absence d'hybridation aux dites copies ADN.

Le produit d'expression qui est détecté peut aussi être un polypeptide qui est le produit de la traduction d'au moins un des transcrits décrits ci-dessus. Auquel cas, on détecte le polypeptide exprimé par mise en contact avec au moins un partenaire de liaison spécifique dudit polypeptide, en particulier un anticorps ou un analogue d'anticorps ou un aptamère. Le partenaire de liaison est de préférence un anticorps, par exemple un anticorps monoclonal ou un anticorps polyclonal hautement purifié ou un analogue d'un anticorps, par exemple une protéine d'affinité aux propriétés compétitives (nanofitine™) .

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Les nanofitines™ sont de petites protéines qui comme les anticorps sont capables de se lier à une cible biologique permettant ainsi de la détecter, de la capturer ou tout simplement de la cibler au sein d'un organisme. On les présente, entre autres, comme des analogues d'anticorps.

Les aptamères sont des oligonucléotides synthétiques capables de fixer un ligand spécifique.

L'invention concerne également l'utilisation d'au moins une séquence d'acide nucléique, isolée, comme marqueur moléculaire pour le diagnostic *in vitro* du cancer de l'ovaire, caractérisée en ce que ladite séquence d'acide nucléique consiste en :
(i) au moins une séquence ADN choisie parmi les séquences SEQ ID NOs :2, 3, 6, 9, 10, 11, 20, 23, 24, 25, 26, 30, 34, 59 et 139, et en particulier choisies parmi les séquences identifiées en SEQ ID NOs : 2, 3 et 6, ou
(ii) au moins une séquence ADN complémentaire d'une séquence choisie parmi les séquences SEQ ID NOs :2, 3, 6, 9, 10, 11, 20, 23, 24, 25, 26, 30, 34, 59 et 139, et en particulier choisies parmi les séquences identifiées en SEQ ID NOs : 2, 3 et 6, ou
(iii) au moins une séquence ADN qui présente au moins 99% d'identité, de préférence au moins 99,5% d'identité et avantageusement au moins 99,6% ou au moins 99,7% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité, de préférence au moins 99,5% d'identité et avantageusement au moins 99,6% ou au moins 99,7% d'identité avec une séquence telle que définie en (i).

Dans un mode de réalisation, on utilise en outre au moins une séquences d'acide nucléique qui consiste en :
(i) au moins une séquence ADN choisie parmi les séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510 et en particulier les séquences SEQ ID Nos : 1, 17 et 29, ou encore au moins une séquence ADN choisie parmi les séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510 et en particulier parmi les séquences SEQ ID Nos : 1, 17 et 29, ou
(ii) au moins deux séquences ADN respectivement complémentaires d'au moins deux séquences telles que définies en (i),
(iii) au moins deux séquences ADN qui présentent respectivement au moins 99% d'identité, de préférence au moins 99,5% d'identité et avantageusement au moins 99,6% ou au moins 99,7% d'identité avec deux séquences telles que définies en (i) et (ii), ou
(iv) au moins deux séquences ARN qui sont respectivement le produit de transcription de deux séquences choisies parmi les séquences telles que définies en (i), ou
(v) au moins deux séquences ARN qui sont le produit de transcription de deux séquences choisies parmi les séquences qui présentent au moins 99% d'identité, de préférence au moins 99,5% d'identité et avantageusement au moins 99,6% ou au moins 99,7% d'identité avec les séquences telles que définies en (i).

L'invention concerne encore un procédé pour évaluer l'efficacité d'un traitement du cancer de l'ovaire qui comprend un étape d'obtention d'une série d'échantillons biologiques, une étape de détection d'au moins un produit d'expression d'au moins une séquence d'acide nucléique dans ladite série d'échantillons biologiques, ladite séquence d'acide nucléique étant choisie parmi les séquences identifiées en SEQ ID NOs : 2, 3, 6, 9, 10, 11, 20, 23, 24, 25, 26, 30, 34, 59 et 139, ou des séquences qui présentent au moins 99% d'identité, de préférence au moins 99,5% d'identité et avantageusement au moins 99,6% ou au moins 99,7% d'identité avec les séquences identifiées en SEQ ID NOs2, 3, 6, 9, 10, 11, 20, 23, 24, 25, 26, 30, 34, 59 et 139. De préférence, on détecte en outre le produit d'expression d'au moins une séquence d'acide nucléique, de préférence d'au moins deux séquences d'acides nucléiques ou de trois séquences d'acide nucléique, lesdites séquences d'acide nucléiques étant choisies dans le groupe de séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510, et de préférence les séquences SEQ ID NOs 1, 17 et 29 ou dans le groupe des séquences qui présentent au moins 99% d'identité, de préférence au moins 99,5% d'identité et avantageusement au moins 99,6% ou au moins 99,7% d'identité avec les séquences identifiées en SEQ ID NOs 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510, et de préférence les séquences SEQ ID NOs : 1, 17 et 29.

Par échantillon biologique on entend un tissu, un fluide, des composants desdits tissu et fluide, tels que des cellules ou des corps apoptotiques, des vésicules excrétées, comprenant notamment des exosomes et des microvésicules. A titre d'exemple, l'échantillon biologique peut être issu d'une biopsie de l'ovaire réalisée au préalable chez un patient suspecté d'être atteint d'un cancer de l'ovaire ou être issu d'une biopsie pratiquée sur un organe autre que l'ovaire chez un patient présentant des métastases. Dans ce deuxième cas, quand le changement d'expression de l'acide nucléique (marqueur moléculaire) est spécifique de l'organe ovaire, il est possible de remonter au cancer primaire, c'est à dire au cancer de l'ovaire. L'échantillon biologique peut être également un fluide biologique, tel que du sang ou une fraction sanguine (sérum, plasma), de l'urine, de la salive, du liquide céphalo-rachidien, de la lymphe, du lait maternel, du sperme, de même que des composants desdits fluides, en particulier des vésicules excrétées telles que définies ci-dessus. Par exemple, la détection d'un transcrit spécifique du tissu ovaire dans un exosome ou une microvésicule, originaire d'une cellule épithéliale, signe soit la présence d'un cancer primaire, soit des métastases, sans qu'il soit nécessaire de faire un prélèvement au niveau de l'organe.

### Figures :

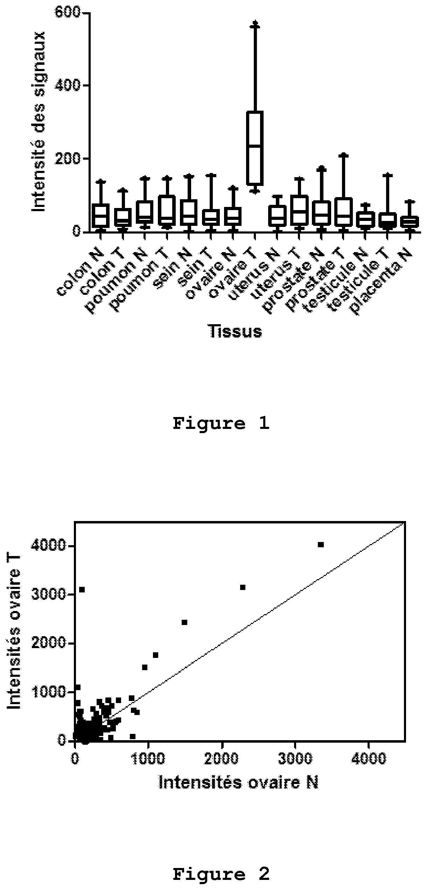

Les figures 1 et 2 représentent le différentiel d'expression observé dans le cancer de l'ovaire pour un ensemble de séquences HERV. Plus précisément, la figure 1 (clustering) regroupe de manière exploratoire les éléments HERV qui ont un tropisme d'expression associé au cancer de l'ovaire par rapport à l'ensemble des tissus contrôles, et la figure 2 montre les différences statistiques d'expression d'éléments HERV entre ovaire normal et ovaire tumoral.
Les figures 3 et 4 montrent la détection de séquences HERV dans deux fluides biologiques : les urines et les sérums.

### Exemples :

### Exemple 1 : Identification de séquences HERV présentant un différentiel d'expression dans le cancer de l'ovaire

### Méthode :

L'identification de séquences HERV présentant un différentiel d'expression dans le cancer de l'ovaire repose sur la conception et l'utilisation d'une puce à ADN haute densité au format GeneChip, dénommée HERV-V2, conçue par les inventeurs et dont la fabrication a été sous-traitée à la société Affymetrix. Cette puce contient des sondes qui correspondent à des séquences HERV distinctes au sein du génome humain. Ces séquences ont été identifiées à partir d'un ensemble de références prototypiques découpées en régions fonctionnelles (LTR, *gag, pol* et env) puis, par une recherche de similarité à l'échelle du génome humain entier (NCBI 36/hg18), 10 035 loci HERV distincts ont été identifiés, annotés, et finalement regroupés dans une banque de données nommée HERVgDB3.

Les sondes entrant dans la composition de la puce ont été définies à partir de HERVgDB3 et sélectionnées en appliquant un critère de spécificité d'hybridation, dont le but est d'exclure du procédé de création les sondes présentant un risque d'hybridation élevé avec une cible non recherchée. Pour cela, les séquences de HERVgDB3 ont d'abord été segmentées par ensemble de 25 nucléotides (25-mers) chevauchants, aboutissant à un ensemble de sondes candidates. Le risque d'hybridation aspécifique a ensuite été évalué pour chaque sonde candidate en réalisant des alignements sur l'ensemble du génome humain à l'aide de l'algorithme KASH (2). Un score expérimental sanctionne le résultat de l'hybridation, addition de l'impact du nombre, du type et de la position des erreurs dans l'alignement. La valeur de ce score est corrélée au potentiel d'hybridation cible/sonde. La connaissance de tous les potentiels d'hybridation d'une sonde candidate sur l'ensemble du génome humain permet d'évaluer sa spécificité de capture. Les sondes candidates qui présentent une bonne affinité de capture sont conservées puis regroupées en « probesets » et enfin synthétisées sur la puce HERV-V2.

Les échantillons analysés à l'aide de la puce haute densité HERV-V2 correspondent à des ARN extraits de tumeurs et aux ARN extraits des tissus sains adjacents à ces tumeurs. Les tissus analysés sont l'ovaire, avec en contrôles le sein, le côlon, l'utérus, la prostate, le poumon, le testicule et le placenta. Dans le cas du placenta, seuls des tissus sains ont été utilisés. Pour chaque échantillon, 50ng d'ARN ont servi à la synthèse de cDNA en utilisant le protocole d'amplification connu sous le nom de WTO. Le principe de l'amplification WTO est le suivant : des amorces aléatoires, ainsi que des amorces ciblant l'extrémité 3' du transcrit ARN, sont ajoutées, avant une étape de transcription inverse suivie d'une amplification linéaire et simple brin désignée SPIA. Les cDNA sont ensuite dosés, caractérisés et purifiés, puis 2µg sont fragmentés, marqués à la biotine en extrémité 3' par l'action de l'enzyme *terminal transferase.* Le produit cible ainsi préparé est mélangé à des oligonucléotides de contrôle, puis l'hybridation est réalisée selon le protocole recommandé par la société Affymetrix. Les puces sont alors révélées et lues pour acquérir l'image de leur fluorescence. Un contrôle qualité se basant sur les contrôles standards est réalisé, et un ensemble d'indicateurs (MAD, MAD-Med plots, RLE) servent à exclure les puces non conformes à une analyse statistique.

L'analyse des puces consiste d'abord en un pré-traitement des données par l'application d'une correction du bruit de fond basée sur l'intensité des signaux des sondes tryptophanes, suivie d'une normalisation RMA (3) basée sur la méthode des quantiles. Puis une double correction des effets liés aux lots d'expériences est réalisée en appliquant la méthode COMBAT (4) afin de garantir que les différences d'expression observées sont d'origine biologique et non techniques. A ce stade, une analyse exploratoire des données est conduite à l'aide d'outils de regroupement de données par partitionnement euclidien (*clustering*), et enfin une analyse statistique utilisant la procédure SAM (5) suivie d'une correction par le taux de faux positif (6) et d'une suppression des valeurs inférieures à 2⁶ est appliquée pour la recherche de séquences présentant un différentiel d'expression entre l'état normal et l'état tumoral d'un tissu.

### Résultats :

Le traitement des données générées par l'analyse des puces à ADN HERV-V2 à l'aide de cette méthode a permis d'identifier un ensemble de « probesets » présentant une différence d'expression statistiquement significative entre l'ovaire normal et l'ovaire tumoral. Les résultats du *clustering* ainsi que la recherche d'expression différentielle au sein des échantillons contrôles a par ailleurs mis en évidence des éléments HERV dont l'expression différentielle est spécifiquement associée à l'ovaire tumoral.

Les séquences nucléotidiques des éléments HERV présentant un différentiel d'expression dans l'ovaire tumoral sont identifiées par les SED ID N°1 à 510, la localisation chromosomique de chaque séquence est donnée dans le référentiel NCBI 36/hg18, et la mention « tissu cible » (une croix) pointe les éléments dont l'expression différentielle n'a été observée que dans la comparaison entre ovaire normal et ovaire tumoral (par rapport aux comparaisons au sein des tissus contrôles). Une valeur indicative du ratio d'expression entre état normal et état tumoral est également communiquée, et sert à ordonner les séquences dans un soucis de présentation uniquement.

### Exemple 2 : Détection de séquences HERV dans les fluides biologiques

### Principe :

Les inventeurs ont montré que des séquences HERV sont détectées dans les fluides biologiques, ce qui permet entre autres de caractériser un cancer de l'ovaire par recours à une détection à distance de l'organe primaire. Une étude a été menée sur 20 échantillons d'urine et 38 échantillons de sérum provenant d'individus différents.

Les sérums et les urines ont été centrifugés dans les conditions suivantes :
Sérums : 500 g pendant 10 minutes à 4°C. Le surnageant a été récupéré et centrifugé de nouveau à 16500 g pendant 20 minutes à 4°C. Le surnageant de cette deuxième centrifugation, dépourvu de cellules, mais comprenant encore des exosomes, des microvésicules, des acides nucléiques, des protéines, a été analysé sur des puces. La puce est la puce HERV-V2 utilisée selon les modalités précédemment décrites.

Urines : après recueil, centrifugation à 800 g pendant 4 minutes à 4°C. Le culot a été récupéré avec du RNA protect cell reagent™. Puis, centrifugation à 5000 g pendant 5 minutes avant ajout du tampon de lyse sur le culot. La puce est la puce HERV-V2 utilisée selon les modalités précédemment décrites.

### Résultats :

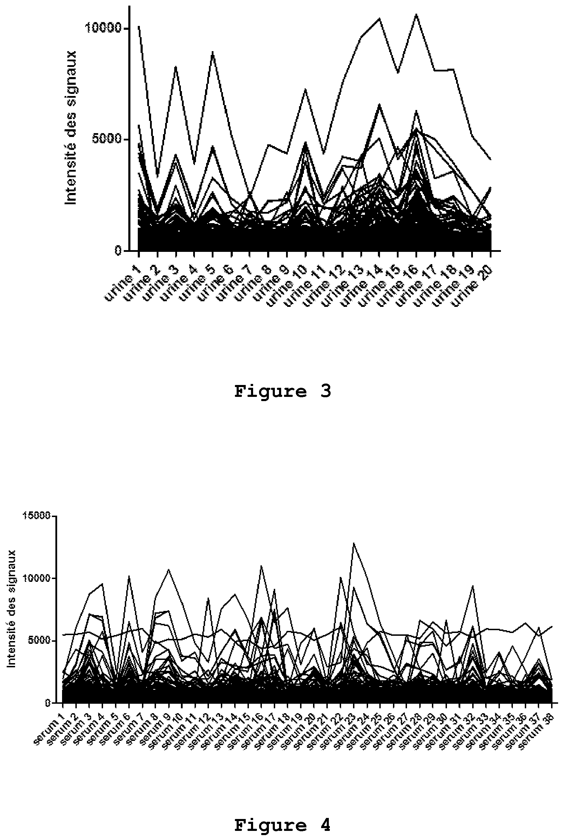

Un nombre important de signaux positifs, incluant les signaux d'expression correspondant aux séquences listées dans le tableau ci-dessus, a été détecté à la fois dans les surnageants de sérums et dans les culots cellulaires provenant d'urines, comme illustré dans les figures 3 et 4. Ceci confirme que les fluides biologiques, en particulier le sérum et l'urine, sont une source utilisable de matériel biologique pour la détection de séquences HERV. Il est communément accepté que le seuil de positivité est de l'ordre de 2⁶ soit 64.

### Références bibliographiques

1. Nickerson,D.A., Taylor,S.L., Weiss,K.M., Clark,A.G., Hutchinson,R.G., Stengard,J., Salomaa,V., Vartiainen,E., Boerwinkle,E. and Sing,C.F. (1998) DNA sequence diversity in a 9.7-kb région of the human lipoprotein lipase gene. Nat. Genet., 19, 233-240.
2. Navarro,G. and Raffinot,M. (2002) Flexible Pattern Matching in Strings: Practical On-Line Search Algorithms for Texts and Biological Sequences. Cambridge University Press.
3. Irizarry,R.A., Hobbs,B., Collin,F., Beazer-Barclay,Y.D., Antonellis,K.J., Scherf,U. and Speed,T.P. (2003) Exploration, normalization, and summaries of high density oligonucleotide array probe level data. Biostatistics (Oxford, England), 4, 249-264.
4. Johnson,W.E., Li,C. and Rabinovic,A. (2007) Adjusting batch effects in microarray expression data using empirical Bayes methods. Biostatistics (Oxford, England), 8, 118-127.
5. Tusher,V.G., Tibshirani,R. and Chu,G. (2001) Significance analysis of microarrays applied to the ionizing radiation response. Proceedings of the National Academy of Sciences of the United States of America, 98, 5116-5121.
6. Storey,J.D. and Tibshirani,R. (2003) Statistical significance for genomewide studies. Proceedings of the National Academy of Sciences of the United States of America, 100, 9440-9445.

## Revendications

1. Procédé pour le diagnostic, *in vitro,* spécifique du cancer de l'ovaire dans un échantillon biologique prélevé chez un patient qui comprend une étape de détection d'au moins un produit d'expression d'au moins une séquence d'acide nucléique, ladite séquence d'acide nucléique étant choisie parmi les séquences identifiées en SEQ ID NOs : 2, 3, 6, 9, 10, 11, 20, 23, 24, 25, 26, 30, 34, 59 et 139 ou parmi les séquences qui présentent au moins 99% d'identité avec une des séquences identifiées en SEQ ID NOs: 2, 3, 6, 9, 10, 11, 20, 23, 24, 25, 26, 30, 34, 59 et 139.

2. Procédé selon la revendication 1, dans lequel on détecte en outre le produit d'expression d'au moins une séquence d'acide nucléique choisie dans le groupe de séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510 ou parmi les séquences qui présentent au moins 99% d'identité avec les séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510.

3. Procédé selon la revendication 2, dans lequel est détecté le produit d'expression d'au moins deux séquences d'acide nucléique, de préférence de trois séquences d'acide nucléique, choisies dans le groupe de séquences identifiées en SEQ ID NOs 1, 17 et 29, ou parmi les séquences qui présentent au moins 99% d'identité avec les séquences identifiées en SEQ ID NOs : 1, 17 et 29.

4. Procédé selon la revendication 1, dans lequel est détecté le produit d'expression d'au moins deux séquences d'acide nucléique choisies dans le groupe constitué par les séquences identifiées en SEQ ID NOs : 2, 3, 6, 9, 10, 11, 20, 23, 24, 25, 26, 30, 34, 59 et 139 ou choisies parmi les séquences qui présentent au moins 99% d'identité avec une des séquences identifiées en SEQ ID NOs : 2, 3, 6, 9, 10, 11, 20, 23, 24, 25, 26, 30, 34, 59 et 139.

5. Procédé selon la revendication 4, dans lequel est détecté le produit d'expression d'au moins deux séquences d'acide nucléique, de préférence de trois séquences d'acide nucléique, choisies dans le groupe de séquences identifiées en SEQ ID Nos : 2, 3 et 6, ou parmi les séquences qui présentent au moins 99% d'identité avec les séquences identifiées en SEQ ID NOs : 2, 3 et 6.

6. Procédé selon l'une quelconque des revendications précédentes, dans lequel le produit d'expression détecté est au moins un transcrit ARN ou au moins un polypeptide.

7. Procédé selon la revendication 6, **caractérisé en ce que** le transcrit ARN est au moins un ARNm.

8. Procédé selon la revendication 6 ou 7, dans lequel le transcrit ARN, en particulier l'ARNm est détecté par hybridation, par amplification ou par séquençage.

9. Procédé selon la revendication 7, dans lequel l'ARNm est mis en contact avec au moins une sonde et/ou au moins une amorce dans des conditions prédéterminées permettant l'hybridation et en ce qu'on détecte la présence ou l'absence d'hybridation à l'ARNm.

10. Procédé selon la revendication 7, **caractérisé en ce qu'**on prépare des copies ADN de l'ARNm, on met en contact les copies ADN avec au moins une sonde et/ou au moins une amorce dans des conditions prédéterminées permettant l'hybridation et **en ce qu'**on détecte la présence ou l'absence d'hybridation aux dites copies ADN.

11. Procédé selon la revendication 6, dans lequel on détecte le polypeptide exprimé par mise en contact avec au moins un partenaire de liaison spécifique dudit polypeptide, en particulier un anticorps ou un analogue d'anticorps, une protéine d'affinité ou un aptamère.

12. Utilisation d'au moins une séquence d'acide nucléique, isolée, comme marqueur moléculaire pour le diagnostic in vitro, spécifique du cancer de l'ovaire, **caractérisée en ce que** la séquence d'acide nucléique consiste en :
(i) au moins une séquence ADN choisie parmi les séquences SEQ ID NOs : 2, 3, 6, 9, 10, 11, 20, 23, 24, 25, 26, 30, 34, 59 et 139 et en particulier choisies parmi les séquences identifiées en SEQ ID NOs : 2, 3 et 6 ;
(ii) au moins une séquence ADN complémentaire d'au moins une séquence telles que définie en (i),
(iii) au moins une séquence ADN qui présente au moins 99% d'identité, de préférence au moins 99,5% d'identité et avantageusement au moins 99,6% ou au moins 99,7% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité, de préférence au moins 99,5% d'identité et avantageusement au moins 99,6% ou au moins 99,7% d'identité avec les séquences telles que définies en (i).

13. Utilisation selon la revendication 12, **caractérisée en ce qu'**on utilise en outre une séquence d'acide nucléique qui consiste en :
(i) au moins une séquence ADN choisie parmi les séquences identifiées en SEQ ID NOs : 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 et 510 et en particulier les séquences SEQ ID Nos : 1, 17 et 29,, ou
(ii) au moins une séquence ADN complémentaire d'au moins une séquence telles que définie en (i),
(iii) au moins une séquence ADN qui présente au moins 99% d'identité, de préférence au moins 99,5% d'identité et avantageusement au moins 99,6% ou au moins 99,7% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité, de préférence au moins 99,5% d'identité et avantageusement au moins 99,6% ou au moins 99,7% d'identité avec les séquences telles que définies en (i).

14. Procédé pour évaluer l'efficacité d'un traitement du cancer de l'ovaire qui comprend une étape de détection d'au moins un produit d'expression d'au moins une séquence d'acide nucléique choisie parmi les séquences identifiées en SEQ ID NOs : 2, 3, 6, 9, 10, 11, 20, 23, 24, 25, 26, 30, 34, 59 et 139 ou parmi les séquences qui présentent au moins 99% d'identité respectivement avec les séquences identifiées en SEQ ID NOs : 2, 3, 6, 9, 10, 11, 20, 23, 24, 25, 26, 30, 34, 59 et 139.

15. Procédé selon la revendication 14, dans lequel on détecte en outre le produit d'expression d'au moins une séquence d'acide nucléique choisie dans le groupe de séquences telles que définies dans la revendication 2.

## Patentansprüche

1. Verfahren zur spezifischen *in vitro*-Diagnose von Eierstockkrebs in einer von einem Patienten entnommenen biologischen Probe, das einen Detektionsschritt von mindestens einem Expressionsprodukt von mindestens einer Nukleinsäuresequenz umfasst, wobei die Nukleinsäuresequenz gewählt ist aus den Sequenzen, identifiziert durch SEQ ID NO: 2, 3, 6, 9, 10, 11, 20, 23, 24, 25, 26, 30, 34, 59 und 139 oder aus den Sequenzen, die mindestens 99 % Identität aufweisen mit einer der Sequenzen, identifiziert durch SEQ ID NO: 2, 3, 6, 9, 10, 11, 20, 23, 24, 25, 26, 30, 34, 59 und 139.

2. Verfahren gemäß Anspruch 1, wobei des Weiteren das Expressionsprodukt von mindestens einer Nukleinsäuresequenz, gewählt aus der Gruppe von Sequenzen, identifiziert durch SEQ ID NO: 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 und 510 oder aus den Sequenzen, die mindestens 99 % Identität aufweisen mit den Sequenzen, identifiziert durch SEQ ID NO: 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 und 510 detektiert wird.

3. Verfahren gemäß Anspruch 2, wobei das Expressionsprodukt von mindestens zwei Nukleinsäuresequenzen, vorzugsweise von drei Nukleinsäuresequenzen, gewählt aus der Gruppe von Sequenzen, identifiziert durch SEQ ID NO 1, 17 und 29, oder aus den Sequenzen, die mindestens 99 % Identität aufweisen mit den Sequenzen, identifiziert durch SEQ ID NO: 1, 17 und 29, detektiert wird.

4. Verfahren gemäß Anspruch 1, wobei das Expressionsprodukt von mindestens zwei Nukleinsäuresequenzen, gewählt aus der Gruppe, bestehend aus den Sequenzen, identifiziert durch SEQ ID NO: 2, 3, 6, 9, 10, 11, 20, 23, 24, 25, 26, 30, 34, 59 und 139 oder gewählt aus den Sequenzen, die mindestens 99 % Identität aufweisen mit einer der Sequenzen, identifiziert durch SEQ ID NO: 2, 3, 6, 9, 10, 11, 20, 23, 24, 25, 26, 30, 34, 59 und 139, detektiert wird.

5. Verfahren gemäß Anspruch 4, wobei das Expressionsprodukt von mindestens zwei Nukleinsäuresequenzen, vorzugsweise von drei Nukleinsäuresequenzen, gewählt aus der Gruppe von Sequenzen, identifiziert durch SEQ ID NO: 2, 3 und 6 oder aus den Sequenzen, die mindestens 99 % Identität aufweisen mit den Sequenzen, identifiziert durch SEQ ID NO: 2, 3 und 6, detektiert wird.

6. Verfahren gemäß einem der vorstehenden Ansprüche, wobei das detektierte Expressionsprodukt mindestens eine transkribierte RNA oder mindestens ein Polypeptid ist.

7. Verfahren gemäß Anspruch 6, **dadurch gekennzeichnet, dass** die transkribierte RNA mindestens eine mRNA ist.

8. Verfahren gemäß Anspruch 6 oder 7, wobei die transkribierte RNA, insbesondere die mRNA, durch Hybridisierung, durch Amplifikation oder durch Sequenzierung detektiert wird.

9. Verfahren gemäß Anspruch 7, wobei die mRNA unter vorgegebenen Bedingungen, die die Hybridisierung erlauben, mit mindestens einer Sonde und/oder mindestens einem Primer in Kontakt gebracht wird, und die Gegenwart oder Abwesenheit von Hybridisierung an der mRNA detektiert wird.

10. Verfahren gemäß Anspruch 7, **dadurch gekennzeichnet, dass** DNA-Kopien der mRNA vorbereitet werden, die DNA-Kopien mit mindestens einer Sonde und/oder mindestens einem Primer unter vorgegebenen Bedingungen, die die Hybridisierung erlauben, in Kontakt gebracht werden und dadurch, dass die Gegenwart oder die Abwesenheit von Hybridisierung der DNA-Kopien detektiert wird.

11. Verfahren gemäß Anspruch 6, wobei das Polypeptid, das durch in Kontakt bringen mit mindestens einem spezifischen Bindungspartner des Polypeptids, insbesondere einem Antikörper oder einem Antikörperanalogon, einem Affinitätsprotein oder einem Aptamer exprimiert wird, detektiert wird.

12. Verwendung von mindestens einer isolierten Nukleinsäuresequenz als molekularem Marker für die spezifische in vitro-Diagnose von Eierstockkrebs, **dadurch gekennzeichnet, dass** die Nukleinsäuresequenz aus Folgendem besteht:
(i) mindestens einer DNA-Sequenz, gewählt aus den Sequenzen SEQ ID NO: 2, 3, 6, 9, 10, 11, 20, 23, 24, 25, 26, 30, 34, 59 und 139 und insbesondere gewählt aus den Sequenzen, identifiziert durch SEQ ID NO: 2, 3 und 6;
(ii) mindestens einer komplementären DNA-Sequenz von mindestens einer Sequenz wie in (i) definiert,
(iii) mindestens einer DNA-Sequenz, die mindestens 99 % Identität, vorzugsweise mindestens 99,5 % Identität und vorteilhafterweise mindestens 99,6 % oder mindestens 99,7 % Identität mit einer Sequenz wie in (i) und (ii) definiert, aufweist, oder
(iv) mindestens einer RNA-Sequenz, die das Transkriptionsprodukt einer Sequenz ist, gewählt aus den Sequenzen wie in (i) definiert, oder
(v) mindestens einer RNA-Sequenz, die das Transkriptionsprodukt einer Sequenz ist, gewählt aus den Sequenzen, die mindestens 99 % Identität, vorzugsweise mindestens 99,5 % Identität und vorteilhafterweise mindestens 99,6 % oder mindestens 99,7 % Identität mit den Sequenzen wie in (i) definiert, aufweisen.

13. Verwendung gemäß Anspruch 12, **dadurch gekennzeichnet, dass** des Weiteren eine Nukleinsäuresequenz verwendet wird, die aus Folgendem besteht:
(i) mindestens einer DNA-Sequenz, gewählt aus den Sequenzen, identifiziert durch SEQ ID NO: 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 und 510 und insbesondere den Sequenzen SEQ ID No: 1, 17 und 29,, oder
(ii) mindestens einer komplementären DNA-Sequenz von mindestens einer Sequenz wie in (i) definiert,
(iii) mindestens einer DNA-Sequenz, die mindestens 99 % Identität, vorzugsweise mindestens 99,5 % Identität und vorteilhafterweise mindestens 99,6 % oder mindestens 99,7 % Identität mit einer Sequenz wie in (i) und (ii) definiert, aufweist, oder
(iv) mindestens einer RNA-Sequenz, die das Transkriptionsprodukt einer Sequenz ist, gewählt aus den Sequenzen wie in (i) definiert, oder
(v) mindestens einer RNA-Sequenz, die das Transkriptionsprodukt einer Sequenz ist, gewählt aus den Sequenzen, die mindestens 99 % Identität, vorzugsweise mindestens 99,5 % Identität und vorteilhafterweise mindestens 99,6 % oder mindestens 99,7 % Identität mit den Sequenzen wie in (i) definiert, aufweisen.

14. Verfahren zum Bewerten der Wirksamkeit einer Behandlung von Eierstockkrebs, das einen Detektionsschritt von mindestens einem Expressionsprodukt von mindestens einer Nukleinsäuresequenz umfasst, die gewählt ist aus den Sequenzen, identifiziert durch SEQ ID NO: 2, 3, 6, 9, 10, 11, 20, 23, 24, 25, 26, 30, 34, 59 und 139 oder aus den Sequenzen, die mindestens 99 % Identität aufweisen mit jeweils folgenden Sequenzen, identifiziert durch SEQ ID NO: 2, 3, 6, 9, 10, 11, 20, 23, 24, 25, 26, 30, 34, 59 und 139.

15. Verfahren gemäß Anspruch 14, wobei des Weiteren das Expressionsprodukt von mindestens einer Nukleinsäuresequenz detektiert wird, gewählt aus der Gruppe von Sequenzen wie in Anspruch 2 definiert.

## Claims

1. A method for the *in vitro* diagnosis specific to ovarian cancer in a biological sample taken from a patient which comprises a step of detecting at least one expression product of at least one nucleic acid sequence, said nucleic acid sequence being selected from the sequences identified in SEQ ID NOs: 2, 3, 6, 9, 10, 11, 20, 23, 24, 25, 26, 30, 34, 59 and 139, or from the sequences which have at least 99% of identity with one of the sequences identified in SEQ ID NOs: 2, 3, 6, 9, 10, 11, 20, 23, 24, 25, 26, 30, 34, 59 and 139.

2. The method according to claim 1, wherein there is further detected the expression product of at least one nucleic acid sequence, selected from the group of sequences identified in SEQ ID NOS: 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154,155, 156, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 and 510 or from the sequences which have at least 99% of identity with the sequences identified in SEQ ID NOs: 1, 4, 5, 7, 8, 12, 13, 14, 15, 16, 17, 18, 19, 21, 22, 27, 28, 29, 31, 32, 33, 35, 36, 37, 38, 39, 40, 41, 42, 43, 44, 45, 46, 47, 48, 49, 50, 51, 52 , 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122,123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 179, 180, 181, 182, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 and 510.

3. The method according to claim 2, wherein the expression product of at least two nucleic acid sequences, preferably of three nucleic acid sequences, selected from the group of sequences identified in SEQ ID NOs 1, 17 and 29, or from the sequences which have at least 99% of identity with the sequences identified in SEQ ID NOs: 1,17 and 29 is detected.

4. The method according to claim 1, wherein the expression product of at least two nucleic acid sequences selected from the group constituted of the sequences identified in SEQ ID NOs: 2, 3, 6, 9, 10, 11, 20, 23, 24, 25, 26, 30, 34, 59 and 139 or selected from the sequences which have at least 99% of identity with one of the sequences identified in SEQ ID NOs: 2, 3, 6, 9, 10, 11, 20, 23, 24, 25, 26, 30, 34, 59 and 139 is detected.

5. The method according to claim 4, wherein the expression product of at least two nucleic acid sequences, preferably of three nucleic acid sequences, selected from the group of sequences identified in SEQ ID NOs: 2, 3 and 6, or from the sequences which have at least 99% of identity with the sequences identified in SEQ ID NOs: 2, 3 and 6 is detected.

6. The method according to any one of the preceding claims, wherein the detected expression product is at least one RNA transcript or at least one polypeptide.

7. The method according to claim 6, **characterized in that** the RNA transcript is at least one mRNA.

8. The method according to claim 6 or 7, wherein the RNA transcript, in particular the mRNA is detected by hybridization, amplification or sequencing.

9. The method according to claim 7, wherein the mRNA is brought in contact with at least one probe and/or at least one primer under predetermined conditions allowing the hybridization and the presence or the absence of hybridization to the mRNA is detected.

10. The method according to claim 7, **characterized in that** DNA copies of the mRNA are prepared, the DNA copies are brought in contact with at least one probe and/or at least one primer under predetermined conditions allowing the hybridization and **in that** the presence or the absence of hybridization to said DNA copies is detected.

11. The method according to claim 6, wherein the expressed polypeptide is detected by bringing in contact with at least one specific binding partner of said polypeptide, in particular an antibody or an antibody analog, an affinity protein or an aptamer.

12. A use of at least one isolated nucleic acid sequence as a molecular marker for the *in vitro* diagnosis, specific to ovarian cancer, **characterized in that** the nucleic acid sequence consists of:
(i) at least one DNA sequence selected from the sequences SEQ ID NOs: 2, 3, 6, 9, 10, 11, 20, 23, 24, 25, 26, 30, 34, 59 and 139 and in particular selected from the sequences identified in SEQ ID NOs: 2, 3 and 6;
(ii) at least one DNA sequence complementary to at least one sequence as defined in (i),
(iii) at least one DNA sequence which has at least 99% of identity, preferably at least 99.5% of identity and advantageously at least 99.6% or at least 99.7% of identity with a sequence as defined in (i) and (ii), or
(iv) at least one RNA sequence which is the transcription product of a sequence selected from the sequences as defined in (i), or
(v) at least one RNA sequence which is the transcription product of a sequence selected from sequences which have at least 99% of identity, preferably at least 99.5% of identity and advantageously at least 99.6 % or at least 99.7% of identity with the sequences as defined in (i).

13. The use according to claim 12, **characterized in that** a nucleic acid sequence is further used which consists of:
(i) at least one DNA sequence selected from the sequences identified in SEQ ID NOs: 1, 4, 5, 7, 8, 12, 13, 14, 15, 16, 17, 18, 19, 21, 22, 27, 28, 29, 31, 32, 33, 34, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509 and 510 and in particular the sequences SEQ ID NOs:1, 17 and 29, or
(ii) at least one DNA sequence complementary to at least one sequence as defined in (i),
(iii) at least one DNA sequence which has at least 99% of identity, preferably at least 99.5% of identity and advantageously at least 99.6% or at least 99.7% of identity with a sequence as defined in (i) and (ii), or
(iv) at least one RNA sequence which is the transcription product of a sequence selected from the sequences as defined in (i), or
(v) at least one RNA sequence which is the transcription product of a sequence selected from the sequences which have at least 99% of identity, preferably at least 99.5% of identity, and advantageously at least 99.6% or at least 99.7% of identity with the sequences as defined in (i).

14. A method for evaluating the efficiency of ovarian cancer which comprises a step of detecting at least one expression product of at least one nucleic acid sequence selected from the sequences identified in SEQ ID NOs: 2, 3, 6, 9, 10, 11, 20, 23, 24, 25, 26, 30, 34, 59 and 139 or from the sequences which have at least 99% of identity respectively with the sequences identified in SEQ ID NOs: 2, 3, 6, 9, 10, 11, 20, 23, 24, 25, 26, 30, 34, 59 and 139.

15. The method according to claim 14, wherein the expression product of at least one nucleic acid sequence selected from the group of sequences as defined in claim 2 is further detected.
